# FASCICULE DE BREVET EUROPEEN

(11) **EP 3 283 874 B1**
(45) Date de publication et mention de la délivrance du brevet: **23.02.2022**
(21) Numéro de dépôt: 16718297.1
(22) Date de dépôt: 15.04.2016
(51) Int. Cl.: G01N 27/327, A61B 5/145, A61B 5/1486, C12Q 1/00

(54) **ÉLECTRODES DE DÉTECTION ÉLECTROCHIMIQUE IMPLANTABLES**
IMPLANTIERBARE ELEKTRODEN ZUR ELEKTROCHEMISCHEN DETEKTION
IMPLANTABLE ELECTRODES FOR ELECTROCHEMICAL DETECTION

(30) Priorité: 16.04.2015 FR 1553413
(43) Date de publication de la demande: 21.02.2018
(73) Titulaire: Université Claude Bernard Lyon 1, 69622 Villeurbanne Cedex (FR); Institut National des Sciences Appliquées de Lyon, 69621 Villeurbanne (FR); Centre National de la Recherche Scientifique CNRS, 75794 Paris Cedex 16 (FR); Ecole Centrale de Lyon, 69134 Ecully Cedex (FR); Institut National de la Santé et de la Recherche Médicale (I.N.S.E.R.M.), 75654 Paris Cedex 13 (FR)
(72) Inventeur: MARINESCO, Stéphane, 69008 Lyon (FR); SABAC, Andrei, 69001 Lyon (FR); MORENO-VELASQUEZ, Laura, 10115 Berlin (DE)
(74) Mandataire: IPAZ
(86) Numéro de dépôt international: PCT/EP2016/058449
(87) Numéro de publication internationale: WO 2016/166343

(56) Documents cités:
- EP-A1- 1 906 177
- EP-A1- 2 796 093
- US-A1- 2005 019 212
- VASYLIEVA N ET AL: "Simple and non toxic enzyme immobilization onto platinum electrodes for detection of metabolic molecules in the rat brain using silicon micro-needles", PROCEDIA ENGINEERING, vol. 25, 2011, pages 1361-1364, XP028436794, ISSN: 1877-7058, DOI: 10.1016/J.PROENG.2011.12.336 [extrait le 2012-01-08]
- VITTORIO GUARNIERI ET AL: "Platinum metallization for MEMS application", BIOMATTER, vol. 4, no. 1, 17 janvier 2014 (2014-01-17), page e28822, XP055256757, DOI: 10.4161/biom.28822
- Anonymous: "Chemical Vapor Deposition vs. physical Vapor Deposition", , 31 décembre 2008 (2008-12-31), pages 1-1, XP055280767, Extrait de l'Internet: URL:http://documents.indium.com/qdynamo/do wnload.php?docid=1957 [extrait le 2016-06-15]

## Description

La présente invention concerne le domaine de la chimie analytique, et en particulier celui de la détection des molécules biologiques, notamment celles présentes dans le cerveau de l'homme et de l'animal.

Les électrodes de détection électrochimique implantables dans le système nerveux central, et notamment les biocapteurs implantables, sont des outils prometteurs pour permettre un suivi en temps réel du milieu interstitiel du cerveau. En ce qui concerne les biocapteurs, le principe de la détection consiste à immobiliser, sur une microélectrode, une enzyme, par exemple de la classe des oxydases, sélective de la molécule à détecter (l'analyte). Lorsqu'elle est mise en présence de son substrat, l'enzyme le transforme en produisant un composé oxydable tel que le peroxyde d'hydrogène (H₂O₂), à partir de l'oxygène ambiant. Le peroxyde d'hydrogène est ensuite oxydé à la surface de l'électrode et le courant d'oxydation qui en résulte est détecté pour fournir une estimation quantitative de la concentration de la molécule d'intérêt au voisinage du biocapteur. De façon plus générale pour les électrodes de détection électrochimique, l'électrode catalyse une réaction d'oxydo-réduction à sa surface, générant un courant d'oxydoréduction indicateur de la présence et/ou de la quantité d'un composé oxydable ou réductible dans son voisinage.

Actuellement, il est possible de détecter de nombreuses molécules par cette méthode, notamment, dans le cas de biocapteurs implantables le glucose, le lactate, la D-sérine, le glutamate, la choline, l'ATP ou l'adénosine, et dans le cas plus général d'une électrode de détection électrochimique, l'O₂. Les électrodes de détection électrochimique implantables, et notamment les biocapteurs implantables, offrent une résolution temporelle et spatiale inégalée. Ils permettent notamment un contrôle du milieu interstitiel seconde par seconde.

Les biocapteurs implantables les plus courants sont constitués d'un fil de platine recouvert d'un polymère écran permettant de limiter la pénétration de molécules interférentes, et d'une couche d'enzyme (Pernot et al., 2008). Une des principales limitations de ce type de biocapteur est cependant liée à leur caractère invasif. Leur diamètre, généralement compris entre 40 et 200 µm, est en effet plus grand que la distance moyenne entre les capillaires sanguins du cerveau estimée à 15 µm. La rupture de vaisseaux sanguins et le déclenchement de microhémorragies est donc inévitable au moment de l'implantation du biocapteur. Ce problème peut ainsi aboutir à des phénomènes d'inflammation locale, ou de rupture de la barrière hémato-encéphalique qui peuvent impacter la fiabilité des mesures effectuées *in situ.* Le même problème se pose pour des capteurs non-enzymatiques, par exemple pour la mesure de PO₂ dans le tissu cérébral.

Il existe donc un besoin de fabriquer des électrodes de détection électrochimique et notamment des biocapteurs, de faible diamètre, notamment d'un diamètre inférieur à 15 µm, afin de limiter les lésions dans le site d'implantation. Or, il n'existe pas de fil de platine suffisamment rigide pour atteindre de telles tailles. Une des solutions proposées pour surmonter cette limitation consiste à utiliser des fibres de carbone. Malheureusement, le carbone est un matériau peu propice à l'oxydation du peroxyde d'hydrogène. Certains laboratoires ont proposé d'immobiliser la couche d'enzyme sur une fibre de carbone avec un médiateur chimique qui permet d'améliorer l'oxydation du peroxyde d'hydrogène, comme le bleu de prusse, le violet de ruthénium, ou un hydrogel à base d'osmium (Schuvailo et al., 2005). Cependant, ce procédé d'immobilisation est peu stable. L'enzyme n'est retenue que quelques dizaines de minutes au contact de l'électrode et le médiateur chimique s'échappe de la couche d'enzyme. La détection électrochimique n'est en général plus possible après quelques dizaines de minutes.

Une autre solution consiste à métalliser des fibres de carbone. Cette idée a été explorée par certains laboratoires, qui ont déposé une couche de ruthénium ou de rhodium sur la fibre de carbone, par électrodéposition à partir d'une solution standard (Sakslund et al., 1995 ; Wang et al., 1997). Cependant, les dépôts métalliques obtenus de cette manière sont peu stables et perdent leurs propriétés catalytiques rapidement. Ce procédé ne permet donc pas de réaliser des mesures sur de grandes durées et ne permet pas non plus de stockage à long terme des électrodes, pour une utilisation ou une réutilisation ultérieure. Il limite donc considérablement les possibilités d'utilisations des électrodes de détection électrochimique, et notamment des biocapteurs, ainsi que leurs perspectives de commercialisation.

### Objets et résumé de l'invention

Afin de surmonter les inconvénients associés aux électrodes de détection électrochimique de l'art antérieur, les inventeurs de la présente invention proposent d'utiliser un procédé de métallisation par dépôt sous vide. Les inventeurs ont découvert que le dépôt sous vide permettait l'obtention d'une couche métallique mince, de haute pureté, ayant d'excellentes propriétés catalytiques, et une grande stabilité. La couche métallique ainsi déposée conserve en particulier une excellente sensibilité pour la détection du peroxyde d'hydrogène pendant plusieurs mois. Ce procédé de dépôt permet de fabriquer des électrodes de détection électrochimique, et notamment des biocapteurs enzymatiques à détection électrochimique d'un diamètre total inférieur à 15 µm, ce qui réduit considérablement les biais méthodologiques liés à la nature invasive des biocapteurs implantables : lésions cellulaires, microhémorragies, lésions des capillaires cérébraux, inflammation autour du biocapteur. Les électrodes de détection électrochimique selon l'invention, et notamment les biocapteurs, peuvent être utilisées en expérimentation animale, notamment dans le domaine des neurosciences, et sont également compatibles avec une utilisation en clinique humaine pour fournir de nouveaux moyens de neuro-monitoring chez les patients cérébrolésés. Elles sont également utiles pour des méthodes de détection in vitro, par exemple sur des préparations de tissu isolé (tranches de cerveau...), dans lesquelles leur faible taille sera également avantageuse pour limiter les lésions au tissu d'implantation. Elles sont également utilisables sur d'autres préparations biologiques, telles que les cultures cellulaires. De façon plus générale, elles sont utilisables comme outil de détection dans tout dispositif analytique existant, par exemple pour l'analyse de fluides biologiques.

La présente invention concerne donc le dépôt, sur un filament support, d'un matériau apte à catalyser une réaction d'oxydo-réduction de façon à obtenir une électrode de détection électrochimique. Le dépôt est de préférence réalisé par un procédé de dépôt sous vide tel que le dépôt chimique en phase vapeur ou le dépôt physique en phase vapeur. La réaction d'oxydoréduction catalysée est notamment l'oxydation d'un composé oxydable et/ou la réduction d'un composé réductible.

La présente invention porte en particulier sur un procédé de fabrication d'une électrode de détection électrochimique, ledit procédé comprenant le dépôt d'une couche métallique sur un filament support, ledit dépôt étant réalisé par un procédé de dépôt sous vide.

Le procédé de la présente invention est défini dans la revendication 1.

Dans un mode de réalisation, le procédé de dépôt sous vide est un procédé de dépôt chimique en phase vapeur ou un procédé de dépôt physique en phase vapeur, de préférence un procédé de dépôt physique en phase vapeur, en particulier le dépôt physique en phase vapeur sous faisceaux d'électrons (« Electron beam physical vapor déposition » (EBPVD)) ou le dépôt physique en phase vapeur par pulvérisation cathodique.

La couche métallique peut comprendre au moins un métal noble, en particulier le platine, le ruthénium, le rhodium, le palladium ou leur combinaison. La couche métallique peut être apte à catalyser l'oxydation d'un composé oxydable, en particulier H₂O₂. La couche métallique peut également être apte à catalyser la réduction d'un composé réductible, en particulier O₂.

En accord avec la présente invention, le dépôt comprend, avant le dépôt de la couche métallique, le dépôt d'une sous-couche isolante sur le filament support. La couche isolante permet de couper le contact électrique entre la couche métallique et le filament support et de « forcer » la conduction du courant par la couche métallique externe. Le matériau formant la couche isolante a de préférence une résistivité à 25°C d'au moins 10⁶ Ω.cm, en particulier d'au moins 10¹⁰ Ω.cm, plus particulièrement d'au moins 10¹⁴ Ω.cm. Dans l'invention présente la couche isolante est à base d'alumine. La sous-couche isolante est déposée, de préférence, par un procédé de dépôt sous vide, en particulier par dépôt chimique en phase vapeur.

En accord avec la présente invention, le procédé selon l'invention comprend, avant le dépôt de la couche métallique, le dépôt d'une sous-couche d'accroché sur le filament support. La couche d'accroché peut permettre d'augmenter l'adhérence de la couche métallique sur le filament. Lorsque l'électrode de détection électrochimique comprend une sous-couche isolante, la sous-couche d'accroché peut être déposée sur la sous-couche isolante. Selon une mise en œuvre particulière de l'invention, la sous-couche d'accroché est à base de titane ou de chrome. Dans un mode de réalisation, le dépôt de la sous-couche d'accroché est réalisé par un procédé de dépôt sous vide, par exemple l'un des procédés de dépôt sous vide décrits dans la présente demande.

Dans un mode de réalisation, le filament support est une fibre de carbone.

Dans un mode de réalisation, le procédé selon l'invention comprend, après le dépôt de la couche métallique, le dépôt d'un matériau écran apte à limiter la pénétration de molécules interférentes au contact du filament métallisé, en particulier un polymère tel que la poly-m-phénylènediamine, la poly-o-phénylènediamine, le polyphénol ou l'acétate de cellulose. L'électrode fabriquée selon le procédé de l'invention peut-être non-enzymatique. Dans ce cas, le composé oxydé ou réduit à sa surface correspond généralement à l'analyte à détecter. Lorsque l'électrode est non-enzymatique, ledit procédé selon l'invention ne comprend pas d'étape de dépôt d'une couche enzymatique.

Alternativement, l'électrode peut-être enzymatique. En particulier, le procédé selon l'invention peut comprendre, après le dépôt de la couche métallique, le dépôt d'une couche enzymatique apte à catalyser la dégradation d'un analyte en générant un composé oxydable ou un composé réductible, en particulier un composé oxydable. L'électrode de détection électrochimique enzymatique forme un biocapteur.

Dans un mode de réalisation, la couche enzymatique comprend une enzyme de la classe des oxydases, en particulier la glucose oxydase, la lactate oxydase, la D-amino acide oxydase, la glutamate oxydase, la choline oxydase, la xanthine oxydase, la glycerol-3-phosphate oxydase, la cholestérol oxydase ou la glycine oxydase. Lorsqu'un matériau écran a été déposé sur la couche métallique, la couche enzymatique peut-être déposée sur le matériau écran. Selon une mise en œuvre particulière de l'invention, la couche enzymatique est immobilisée sur le filament métallisé, par exemple à l'aide d'un agent chimique de réticulation tel que le glutaraldéhyde, ou le poly(éthylène glycol) diglycidyl ether (PEGDE).

La présente invention porte également sur une électrode de détection électrochimique fabriquée ou susceptible d'être fabriquée selon le procédé tel que décrit dans la présente invention.

En particulier, la présente invention porte sur une électrode de détection électrochimique comprenant ou constituée d'un filament support revêtu d'une couche métallique.

L'électrode de la présente invention est définie dans la revendication 9.

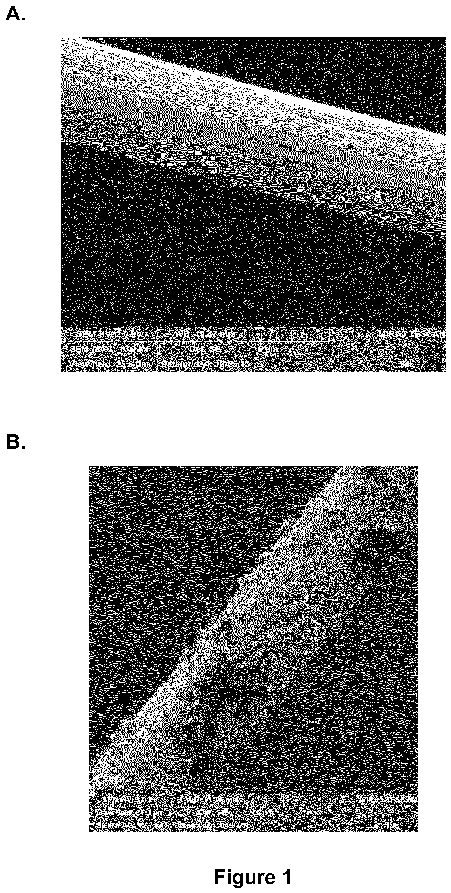

La couche métallique est de préférence déposée par un procédé de dépôt sous vide, ou susceptible d'avoir été déposée par un procédé de dépôt sous vide, en particulier le dépôt chimique en phase vapeur ou le dépôt physique en phase vapeur. Comme le montre la Figure 1, le dépôt sous vide permet d'aboutir à des couches métalliques lisses, d'une uniformité et d'une régularité inégalées, qui contrastent avec les dépôts irréguliers et beaucoup plus épais obtenus par électrodéposition, qui se caractérisent par la présence de grain métalliques d'une épaisseur d'au moins 1 µm (voir également Sakslund et al., 1995, Wang et al., 1997). Les biocapteurs selon l'invention se distinguent donc nettement des biocapteurs utilisant une couche métallique déposée par électrodéposition, tels que décrits dans l'état de la technique.

En accord avec la présente invention, l'électrode comprend une sous-couche isolante entre le filament support et la couche métallique, à base d'alumine.

En accord avec la présente invention, l'électrode comprend une sous-couche d'accroché entre le filament support et la couche métallique. Lorsqu'une sous-couche isolante est présente, la sous-couche d'accroché se situe de préférence entre la sous-couche isolante et la couche métallique. La sous-couche d'accroché peut permettre d'augmenter l'adhérence de la couche métallique sur le filament. Selon une mise en œuvre particulière de l'invention, la sous-couche d'accroché est à base de chrome, de titane, ou de nitrure de titane.

L'électrode fabriquée selon le procédé de l'invention peut-être non-enzymatique, c'est-à-dire non revêtue d'une couche d'enzyme. Alternativement, l'électrode peut être enzymatique. En particulier, la couche métallique peut être revêtue d'une couche enzymatique apte à catalyser la dégradation d'un analyte en générant un composé oxydable ou un composé réductible, en particulier un composé oxydable. L'électrode de détection électrochimique enzymatique forme un biocapteur.

L'électrode peut notamment comprendre, entre la couche métallique et la couche enzymatique, un matériau écran apte à limiter la pénétration de molécules interférentes au contact du filament métallisé, en particulier un polymère tel que la poly-m-phénylènediamine, la poly-o-phénylènediamine, le polyphénol ou l'acétate de cellulose.

Selon une mise en œuvre particulière de l'invention, le diamètre du filament n'excède pas 25 µm, en particulier 20 µm, plus particulièrement 15 µm, et de façon encore plus particulière 10 µm. Dans un autre mode de réalisation, le diamètre du filament est de 1 µm à 25 µm, en particulier de 1 µm à 20 µm, plus particulièrement de 1 µm à 15 µm, et de façon encore plus particulière de 1 µm à 10 µm, ou de 3 µm à 10 µm.

Dans un mode de réalisation, l'épaisseur de la sous-couche d'accroché n'excède pas 50 nm, en particulier 30 nm, et plus particulièrement 20 nm. Dans un autre mode de réalisation, l'épaisseur de la sous-couche d'accroche est de 0,1 nm à 50 nm, en particulier de 0,1 nm à 30 nm, et plus particulièrement de 0,1 à 20 nm. Des couches d'une épaisseur de quelques atomes peuvent être obtenues par la technique d' « atomic layer deposition ».

Dans un mode de réalisation, l'épaisseur de la couche métallique n'excède pas 200 nm, en particulier 150 nm, plus particulièrement 100 nm. Dans un autre mode de réalisation, l'épaisseur de la couche métallique est de 10 nm à 200 µm, en particulier de 10 nm à 150 nm, plus particulièrement de 10 nm à 100 nm. Dans un autre mode de réalisation, l'épaisseur de la couche métallique n'excède pas 10%, en particulier 5%, du diamètre du filament. Dans un autre mode de réalisation, le dépôt de la couche métallique est un dépôt conforme. Le couche métallique est notamment exempte ou sensiblement exempte d'aspérités et/ou irrégularités non présentes à la surface du filament, telles que des grains métalliques déposés à la surface du filament.

Dans un mode de réalisation, l'épaisseur de la couche d'enzyme n'excède pas 10 µm, en particulier 5 µm, de façon plus particulière 3 µm. Dans un autre mode de réalisation, l'épaisseur de la couche d'enzyme est de 1 µm à 10 µm, en particulier de 1 µm à 5 µm, de façon plus particulière de 1 µm à 3 µm. Dans un autre mode de réalisation, l'épaisseur de la couche enzymatique n'excède pas 100%, en particulier 75%, plus particulièrement 50% du diamètre du filament.

Dans un mode de réalisation, l'épaisseur de la couche de matériau écran n'excède pas 100 nm, en particulier 50 nm, de façon plus particulière 20 nm. Dans un autre mode de réalisation, l'épaisseur de la couche de matériau écran est de 1 nm à 100 nm, en particulier de 1 nm à 10 nm, de façon plus particulière de 1 nm à 20 nm ou de 5 nm à 20 nm.

Selon une mise en œuvre particulière de l'invention, le diamètre total de l'électrode, notamment du biocapteur, n'excède pas 100 µm, en particulier 50 µm, de façon plus particulière 15 µm, 14 µm, 13 µm, 12 µm, 11 µm ou 10 µm. Dans un autre mode de réalisation, le diamètre total de l'électrode, notamment du biocapteur est de 5 µm à 15 µm, en particulier de 10 µm à 15 µm, plus particulièrement de 10 µm à 12 µm ou de 12 µm à 15 µm, et encore plus particulièrement de 10 µm à 11 µm, de 11 à 12 µm, de 12 à 13, µm, de 13 à 14 µm ou de 14 à 15 µm.

Dans un mode de réalisation particulier, l'électrode de détection électrochimique selon l'invention comprend :
- un filament support, notamment d'un diamètre de 3 à 10 µm ;
- une couche d'accroché, notamment d'une épaisseur de 0,1 à 20 nm ;
- une couche de platine, notamment d'une épaisseur de 20 à 200 nm .

Dans un mode de réalisation particulier, l'électrode de détection électrochimique forme un biocapteur et comprend :
- un filament support, notamment d'un diamètre de 3 à 10 µm ;
- une couche d'accroché, notamment d'une épaisseur de 0,1 à 20 nm ;
- une couche de platine, notamment d'une épaisseur de 20 à 200 nm ;
- une couche de matériau écran, notamment d'une épaisseur de 5 à 20 nm ;
- une couche d'enzyme, notamment d'une épaisseur de 1 à 5 µm.

Dans un mode de réalisation, le filament support est relié à un moyen de connexion électrique, par exemple un fil métallique tel qu'un fil de cuivre. Le filament support est par exemple collé à la surface dudit fil.

L'isolation de la partie non-sensible du filament et/ou du moyen de connexion électrique peut-être réalisée par insertion du filament dans un capillaire de verre, dont seule dépasse la partie sensible du filament. La jonction entre le filament et le capillaire est alors scellée, par exemple par collage ou par fusion.

Un autre objet de l'invention est un dispositif pour la détection électrochimique d'un analyte, ledit dispositif comprenant :
- une électrode de détection électrochimique, notamment un biocapteur, telle que décrit dans la présente demande ;
- un dispositif d'amplification de courant électrique ;
   et, de façon facultative au moins l'un parmi :
- un dispositif de conversion analogique/numérique ;
- un logiciel d'acquisition et/ou d'exploitation des données de détection ;
- une électrode de référence, en particulier une électrode Ag/AgCl.

Le dispositif de la présente invention est défini dans la revendication 13.

L'invention porte également sur l'utilisation, en tant qu'électrode de détection électrochimique, d'un filament support revêtu d'une couche métallique. En particulier, l'invention porte sur l'utilisation, en tant qu'électrode de détection électrochimique, d'un filament support revêtu d'une couche métallique apte à catalyser l'oxydation d'un composé oxydable. La couche métallique peut être apte à catalyser la réduction d'un composé réductible. La couche métallique peut être déposée par un procédé de dépôt sous vide, ou susceptible d'avoir été déposée par un procédé de dépôt sous vide. Ladite électrode peut être utilisée pour la détection d'un composé oxydable, en particulier le peroxyde d'hydrogène, d'ou d'un analyte susceptible de générer le composé oxydable, par exemple par le biais d'une réaction enzymatique. Dans ce cas, l'électrode peut être recouverte d'une couche d'enzyme pour former un biocapteur électrochimique, en particulier un biocapteur électrochimique selon l'invention. L'électrode peut également être utilisée pour la détection d'un composé réductible, en particulier l'O₂.

Un autre objet de l'invention est un procédé de détection électrochimique d'un analyte dans un milieu de mesure, comprenant:
- le placement d'une électrode de détection électrochimique selon l'invention, en particulier d'un biocapteur selon l'invention, dans le milieu de mesure;
- l'application d'un potentiel de travail à l'électrode de détection électrochimique ou au biocapteur;
- la détection d'un courant d'oxydo-réduction indicateur de la présence et/ou de la concentration dudit analyte dans le milieu de mesure.

Dans un mode de réalisation, le procédé de détection selon l'invention est un procédé in vivo, par exemple mis en œuvre sur un animal de laboratoire, notamment un modèle murin tel que la souris ou le rat, ou mis en œuvre sur l'homme, par exemple pour des applications cliniques, telles que le suivi opératoire ou le diagnostic.

Dans un autre mode de réalisation, le procédé de détection selon l'invention est un procédé in vitro. Le milieu de mesure est par exemple un tissu isolé ou une culture cellulaire. Le milieu de mesure peut également être un fluide biologique, par exemple le milieu interstitiel du cerveau, le sang, le plasma sanguin, le sérum sanguin ou l'urine, ou un dérivé d'un de ces fluides, notamment après traitement et/ou purification.

Dans un mode de réalisation particulier, l'analyte est le glucose, le lactate, le glutamate, la choline, l'acétylcholine, l'adénosine, l'ATP, le cholestérol, la glycine, ou un D-amino acide, en particulier la D-sérine. Dans un autre mode de réalisation, l'analyte est l'O₂.

### Définitions

Un biocapteur est un dispositif intégré capable de fournir des informations spécifiques quantitatives ou semi-quantitatives grâce à un élément de reconnaissance d'origine biologique (tel qu'une enzyme) associé à un élément de transduction (tel qu'une électrode). Dans la présente invention, une électrode de détection électrochimique recouverte d'une couche enzymatique constitue un biocapteur.

Un filament est un élément solide de forme fine et allongée, tel qu'un fil ou une fibre. Le filament, dans la présente invention, sert de support de dépôt, et notamment de métallisation, et constitue, sous forme de filament métallisé, l'électrode de détection électrochimique proprement dite. Le filament peut être constitué de tout matériau susceptible d'être utilisé comme substrat pour le dépôt sous vide d'une couche métallique, notamment le carbone (par exemple sous forme de fibres de carbone) ou un polymère tel que le poly(p-phénylènetéréphtalamide). Il peut être conducteur ou non conducteur. Le filament a de préférence une section transversale circulaire. Il peut être par exemple cylindrique, de forme effilée, ou conique. La partie sensible du filament, c'est-à-dire la partie utile pour la détection du composé oxydable, a de préférence une longueur qui n'excède pas 200 µm, plus particulièrement 150 µm, et de façon plus particulière 100 µm. La longueur de la partie sensible du filament peut notamment être de 10 µm à 200 µm.

Le terme de dépôt sous vide recouvre un ensemble de techniques de dépôt d'une couche mince (un film) sur un substrat solide, ayant en commun d'être réalisées à des pressions plus faibles que la pression atmosphérique, par exemple au moins inférieures à 10⁻¹ torr. Le film est généralement déposé atome par atome ou molécule par molécule. Comme exemple de procédé de dépôt sous vide, on compte notamment le dépôt physique en phase vapeur, ou le dépôt chimique en phase vapeur.

Une oxydase est une enzyme catalysant une réaction d'oxydo-réduction impliquant une molécule de dioxygène (O₂) comme accepteur d'électron, en produisant généralement du peroxyde d'hydrogène (H₂O₂).

Le terme composé oxydable définit tout composé capable d'être transformé dans une réaction d'oxydation électrochimique. Un exemple préféré de composé oxydable, dans la présente invention est le peroxyde d'hydrogène, qui peut être produit par une enzyme de la classe des oxydases.

Une molécule interférente est toute molécule susceptible d'interférer dans la détection de l'analyte. Plus particulièrement, une molécule interférente peut interférer directement dans la détection du composé oxydable au contact du filament métallisé. Une molécule interférente est donc en particulier toute molécule susceptible d'être oxydée au contact du filament métallisé, à l'exception du composé oxydable lui-même, par exemple le peroxyde d'hydrogène (H₂O₂). Les molécules interférentes les plus courantes dans le milieu interstitiel du système nerveux central sont l'acide ascorbique, la dopamine, le DOPA, la noradrénaline, la sérotonine, le 5-HIAA, l'acide urique, et l'acide homovanillique.

Dans la présente invention, les termes couche métallique désignent la couche déposée à la surface d'un filament conducteur ou non conducteur. Ladite couche est à base d'un métal ou de plusieurs métaux capable(s) de catalyser l'oxydation d'un composé oxydable, par exemple le peroxyde d'hydrogène (H₂O₂) et/ou la réduction d'un composé réductible, par exemple l'O₂. La réaction d'oxydation génère un courant d'électrons dont la détection permet une mesure quantitative ou semi-quantitative de la concentration du composé oxydable au voisinage du filament métallisé.

Une sous-couche d'accroche désigne toute couche de matériau déposée sous la couche métallique, et apte à stabiliser le dépôt et/ou améliorer l'adhérence de la couche métallique sur le filament support.

Un matériau écran est un matériau qui permet de limiter la pénétration de molécules interférentes au contact d'une électrode de détection électrochimique. La pénétration du composé oxydable est moins affectée, voire n'est pas affectée du tout par le matériau écran. En d'autres termes, le matériau écran permet d'augmenter la proportion de composé oxydable/molécules interférentes au voisinage de l'électrode. Un matériau écran peut agir par discrimination stérique, c'est-à-dire en limitant la pénétration des molécules ayant une taille supérieure à un seuil de discrimination stérique. C'est notamment le cas des polymères écrans tels que la poly-m-phénylènediamine, la poly-o-phénylènediamine, le polyphénol ou l'acétate de cellulose. Le seuil de discrimination stérique peut être ajusté en variant le degré de polymérisation. Par exemple, le matériau écran peut limiter la pénétration des molécules de taille comparable à l'acide ascorbique, la dopamine, le DOPA, la noradrénaline, la sérotonine, le 5-HIAA, l'acide urique, ou l'acide homovanillique, tout en laissant pénétrer le peroxyde d'hydrogène au voisinage du filament métallisé. Le matériau écran peut également consister en ou comprendre une enzyme de dégradation d'une ou plusieurs molécules interférentes.

Le terme immobilisation désigne tout procédé apte à stabiliser le dépôt de la couche enzymatique sur le biocapteur. L'immobilisation se fait par exemple par fixation covalente à l'aide d'un agent chimique de réticulation (cross-linking) tel que le glutaraldéhyde ou le poly(éthylène glycol) diglycidyl ether (PEGDE).

Les termes détection électrochimique désignent tout procédé de détection qualitative ou quantitative d'une molécule par le biais d'une réaction impliquant la production d'un courant électrique. La détection peut se faire par ampérométrie, par exemple ampérométrie à potentiel constant, ampérométrie puisée, chronoampérométrie, ou voltamétrie, par exemple voltamétrie cyclique, voltamétrie à impulsion, voltamétrie à impulsion différentielle.

Conformément au sens usuel du terme dans les techniques de dépôt sous vide, les termes dépôt conforme désignent le dépôt d'une couche qui épouse la forme des reliefs éventuels existant sur la surface sous-jacente. La couche déposée a donc une épaisseur sensiblement constante sur toute la surface du filament, par exemple, variant de moins de 20%, en particulier moins de 15%, 10% ou 5%.

Sauf indications contraires, toute mesure de taille relative à l'électrode de détection électrochimique, en particulier au biocapteur, dans la présente demande, telle que le diamètre ou la longueur, se rapporte à la partie sensible de l'électrode ou du biocapteur, c'est-à-dire la partie exposée au milieu de mesure, au contact de laquelle ont lieu les réactions d'oxydoréduction servant à la détection du composé oxydable ou réductible.

Pour mieux illustrer la présente invention, on va en décrire ci-après, à titre indicatif et non limitatif, plusieurs modes de réalisations particuliers avec référence aux dessins annexés.

### Brève description des dessins

La Figure 1A est une image en microscopie électronique d'une fibre de carbone de 7 µm métallisée par dépôt physique en phase vapeur, par une couche de platine de 100 nm d'épaisseur.
La Figure 1B est une image en microscopie électronique d'une fibre de carbone de 7 µm métallisée par électrodéposition d'une couche de ruthénium.
La Figure 2 représente un enregistrement d'une durée de 3h de la concentration extracellulaire de glucose dans le cortex d'un rat anesthésié, réalisé à l'aide d'un biocapteur selon l'invention.
A t=60min, l'administration d'insuline provoque la diminution du glucose extracellulaire. A t=112min, l'injection intraveineuse d'un bolus de glucose provoque un rebond rapide de la concentration extracellulaire de glucose.
La Figure 3 représente la relation courant-PO₂ obtenue par mesure ampérométrique de l'oxygène dissous grâce à 6 électrodes de détection électrochimique selon l'invention, composées d'une fibre de carbone platinée.

### Exemples

### Exemple 1 : Matériels et Méthodes

### Préparation des fibres de carbone métallisées

Des fibres de carbone de 7 µm ou 4,8 µm de diamètre (Goodfellow, Lille, France) sont découpées en segments d'environ 5 cm puis collées sur un support métallique à l'aide d'un adhésif en kapton (Radiospares, Beauvais, France) choisi pour sa tenue sans dégazage à haute température (jusqu'à 200°C). Le dépôt s'effectue dans une enceinte sous vide, dans un évaporateur Edwards Auto306 équipé d'un canon à électrons à 5KV. Le support est introduit dans l'évaporateur, un faisceau d'électrons est émis par le filament chauffant sous vide (10⁻⁵ à 10⁻⁷ mbar). L'intensité du courant pendant le dépôt est maintenue autour de 200mA et la température du substrat à moins de 130°C. Le faisceau d'électrons est focalisé au niveau d'un creuset qui contient le matériau à évaporer. La vitesse de dépôt est ajustable par le courant qui traverse le filament. L'épaisseur déposée ainsi que la vitesse de dépôt sont contrôlées avec une mini balance à quartz étalonnée au préalable. Les creusets peuvent être sélectionnés pendant le dépôt par un système automatisé ce qui permet d'enchaîner sous vide le dépôt de plusieurs matériaux de très haute pureté sans remettre l'enceinte à l'air. Le dépôt d'une couche d'accroché, de Ti ou du Cr (en particulier, de 1 à 20 nm) est suivi par le dépôt d'une couche métallique de Pt ou Ru (en particulier de 10 à 200 nm). Les matériaux utilisés sont de très haute pureté (5N). Le support porte-fibre est mis en rotation pendant le dépôt pour obtenir une meilleure homogénéité. Le dépôt se fait sur toute la surface non protégée de la fibre en retournant le support.

A titre de comparaison, des fibres de carbone sont métallisées par électrodéposition d'une couche de ruthénium. Le dépôt de Ru par électrodéposition est effectué par trempage de l'électrode en fibre de carbone dans une solution de standard atomique de Ruthénium (1mg/mL, dans HCI 5%, Sigma-Aldrich, St Quentin Fallavier, France) dont le pH a été ajusté à 2.5 sous un potentiel imposé à -500 mV vs. Ag/AgCl pendant 30 min.

L'uniformité et la régularité de la couche de platine déposée par dépôt physique en phase vapeur (Figure 1A) contraste avec le dépôt irrégulier obtenu par électrodéposition, qui génère des grains d'une épaisseur d'au moins 1 µm à la surface du filament (Figure 1 B). Le dépôt réalisé par dépôt physique en phase vapeur suit la structure striée de la fibre de carbone sous-jacente (Figure 1A)

### Préparation des microélectrodes

Les fibres de carbone métallisées sont ensuite collées individuellement sur un fil de bobinage en cuivre de 0.5 mm de diamètre (Becker, Almacen, Espagne) avec de la peinture d'argent conductrice (Radiospares, Beauvais, France). Elles sont ensuite insérées dans un capillaire en borosilicate étiré et coupé pour laisser passer la fibre métallisée par l'extrémité étirée. Les deux extrémités du capillaire sont ensuite scellées par de la colle époxy (Loctite, Marne la Vallée, France). La fibre de carbone métallisée est ensuite coupée pour ne laisser dépasser que 100 µm hors du capillaire, qui forment la partie sensible de l'électrode.

### Préparation des biocapteurs

Les microélectrodes sont ensuite fonctionnalisées par une première couche de poly-m-phénylènediamine obtenu par électropolymérisation à +700 mV (vs Ag/AgCl) dans une solution de m-phénylènediamine (Sigma-Aldrich, St Quentin-Fallavier, France) 100 mM dans du tampon phosphate salin (PBS, 0.01 M, pH = 7.4). Des biocapteurs enzymatiques peuvent ensuite être préparés à partir d'enzymes de la classe des oxydases immobilisées sur la microélectrode. Le diamètre total de la partie sensible des biocapteurs ainsi obtenus est inférieur à 15 µm, correspondant à l'épaisseur cumulée de la fibre de carbone centrale, de la couche d'accroché, de la couche métallique, de la couche de poly-m-phénylènediamine, et de la couche d'enzyme immobilisée.

Par exemple, des biocapteurs de glucose sont obtenus en déposant sur la pointe de l'électrode une solution visqueuse de glucose oxydase : 60 mg/mL glucose oxydase, 30 mg/mL albumine de sérum bovin (BSA), 1% glycérol et 30 mg/mL de poly(éthylène glycol) diglycidyl ether (PEGDE) dans du PBS 0.01M. L'électrode est ensuite placée à 57°C pendant 2h pour permettre l'immobilisation covalente de l'enzyme par le PEGDE.

Les biocapteurs de lactate sont obtenus de la même manière avec de la lactate oxydase : 60 mg/mL lactate oxydase, 60 mg/mL de BSA, 1 % glycérol et 60 mg/mL PEGDE dans du PBS 0.01 M avec 2h de chauffage à 57 °C.

Les biocapteurs de D-sérine sont obtenus à partir d'une solution de D-amino acide oxydase : 60 mg/mL de D-amino acide oxydase, 30 mg/mL de BSA, 1% glycerol, 10 mg/mL de PEGDE et chauffage pendant 1h45 à 56 °C.

Les biocapteurs de glutamate sont obtenus à partir d'une solution de glutamate oxydase : 100 mg/mL glutamate oxydase, 150 mg/mL BSA, 1% glycérol et 200 mg/mL de PEGDE dans du PBS 0.01 M avec chauffage à 45-47 °C pendant 1h30.

De nombreux autres biocapteurs permettant de détecter la choline, l'acétylcholine, l'adénosine ou l'ATP peuvent aussi être obtenus de cette manière.

### Méthode d'enregistrement et détection

Tous les enregistrements in vitro sont réalisés dans une solution de PBS 0.01 M à pH 7.4. L'électrode de référence est constituée d'un fil d'argent de 0.25 mm de diamètre chloruré dans une solution de KCl 2M. Le potentiel est imposé à +500 mV par rapport à la référence Ag/AgCl grâce à un amplificateur VA-10 (NPI Electronic GmbH, Tamm, Allemagne) et le courant mesuré est numérisé sur une carte d'acquisition NI USB-6221 (National Instruments, Nanterre, France) et analysé sur un logiciel Igor pro 6.1 (Wavemetrics, Lake Oswego, Oregon). La calibration des électrodes, et notamment des biocapteurs se fait par ajouts dosés de solutions standard de concentration connue afin de déterminer la sensibilité des électrodes et des biocapteurs (en densité de courant d'oxydation exprimée en nA.mM⁻¹.cm⁻²) ainsi que leur sélectivité. Dans la présente demande, sauf s'il en est spécifié autrement, la sensibilité des électrodes, et notamment des biocapteurs est exprimée par la médiane et l'intervalle interquartile.

### Exemple 2 : Effet de l'épaisseur de la couche métallique sur la sensibilité à l'H₂O₂

Un dépôt de platine (couche de 20 ou 100 nm) a été réalisé sur une couche d'accroche de titane de 15 nm d'épaisseur. La sensibilité des microélectrodes obtenues avec ces deux épaisseurs de platine était respectivement de 26 (22-32) nA.mM⁻¹.cm⁻² (n=6) et de 145 (97-217) nA.mM⁻¹.cm⁻² (n=69). La couche de platine de 100 nm permet d'obtenir une sensibilité supérieure à la couche de 20 nm.

### Exemple 3 : Effet du matériau utilisé pour la sous-couche d'accroche sur la sensibilité à l'H₂O₂

Les inventeurs ont testé une couche d'accroche de 15 nm de titane ou de chrome en gardant une épaisseur de platine de 100 nm. La sensibilité de 145 (97-217) nA.mM⁻¹.cm⁻² (n=69) obtenue avec la couche de titane est passée à 325 (184 - 629) nA.mM⁻¹.cm⁻² lorsque le Cr était utilisé en sous-couche d'accroché. La couche d'accroche de Cr confère donc une sensibilité de détection supérieure à la couche d'accroche de Ti.

### Exemple 4 : Effet des différentes couches de fonctionnalisation

Les microélectrodes constituées d'un fil de carbone métallisé avec 100 nm de platine sont ensuite fonctionnalisées pour obtenir des biocapteurs de glucose. La première couche de polymère écran en poly-m-phénylènediamine induit une diminution de la sensibilité à l'H₂O₂ qui est passée de 166 (116-217, n=6) à 6 (5-21, n=5) nA.mM⁻¹.cm⁻². Cette sensibilité permet d'obtenir des biocapteurs de glucose d'une sensibilité médiane de 9 (1-28, n=4) nA.mM⁻¹.cm⁻² de glucose.

### Exemple 5 : Stabilité à 6 mois

Deux lots de microélectrodes fabriquées à base d'une fibre de carbone platinée ont été testés à 6 mois d'intervalle. Le premier lot est constitué de fibres de carbone recouvertes d'une couche d'accroche en titane et d'une couche métallique de 100 nm de platine (n=17). Leur sensibilité est passée de 130 (77-156) à 131 (96-166) nA.mM⁻¹.cm⁻². Le deuxième lot est constitué de fibres de carbone recouvertes d'une couche d'accroche en chrome et d'une couche métallique de platine de 100 nm (n=13). En revanche, dans ce lot, la sensibilité est passée de 413 (305-809) à 64 (32-91) nA.mM⁻¹.cm⁻².

La bonne stabilité de la couche métallique, et le maintien de bonnes propriétés catalytiques au bout de plusieurs mois, sont particulièrement surprenants pour un dépôt en phase vapeur sur une surface non plane, telle que celle des fibres de carbone.

La stabilité de la couche métallique étant déterminante dans la stabilité globale de l'électrode, et notamment du biocapteur enzymatique, et le maintien à long terme d'une sensibilité acceptable, ces résultats permettent de prédire une excellente stabilité globale du biocapteur enzymatique. En effet, la stabilité d'une couche d'enzyme peut atteindre plusieurs mois. Par exemple, une couche de D-amino acide oxydase immobilisée par le glutaraldéhyde a pu être conservée 3 mois à -20 °C sans perte significative d'activité (Pernot et al, 2008).

### Exemple 6 : Détection in vivo des concentrations de glucose

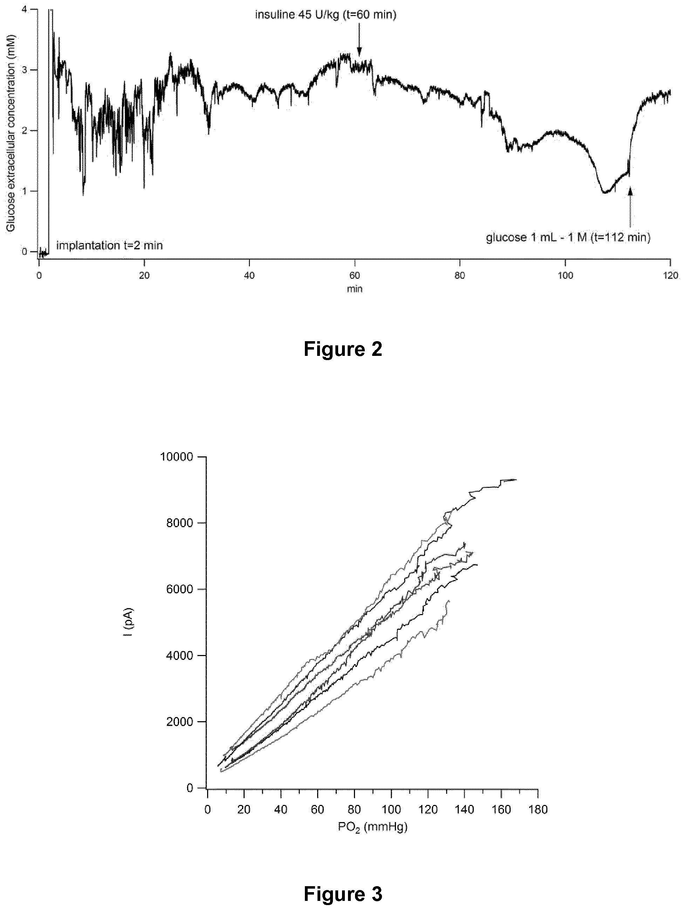

Un biocapteur à glucose de diamètre inférieur à 15 µm est implanté in vivo dans le cortex de rats anesthésiés. Ce biocapteur permet de suivre la concentration extracellulaire de glucose pendant plus de 3 heures dans un protocole consistant à administrer 45 U/kg d'insuline pour faire baisser la concentration de glucose cérébrale, puis à délivrer une dose de 1 mL de glucose à 1 M pour induire une augmentation rapide du glucose intracérébral (Figure 2).

### Exemple 7 : Dépôt chimique en phase vapeur de couches isolantes

Les inventeurs ont déposé, par Atomic Layer Déposition (ALD), une sous-couche isolante d'alumine (Al₂O₃) entre la fibre de carbone support, et la couche de platine. La couche isolante permet de couper le contact électrique entre la couche métallique et le filament support et de « forcer » la conduction du courant par la couche métallique externe. L'Atomic Layer Déposition (ALD) est un procédé de dépôt de type CVD (Chemical Vapor Déposition) permettant d'obtenir des couches minces et ultra-minces parfaitement conformes sur des surfaces à géométrie complexe (par exemple surfaces texturées ou poreuses). La couche mince est le résultat d'un empilement de mono-couches atomiques obtenues par la réaction à la surface de deux précurseurs chimiques envoyés alternativement dans la chambre de dépôt sous vide. La réaction à la surface peut être activée par voie thermique (ex 200-400°C pour l'ALD thermique) ou plasma (PE-ALD). Le PEALD permet de baisser considérablement la température de dépôt (<100°C). L'ALD permet d'obtenir des couches d'oxyde possédant d'excellentes qualités isolantes (Al₂O₃, HfO₂...) ou des couches métalliques (Pt, Ru, Pd, Ir....).

Les microélectrodes avec sous-couche isolante d'alumine ont une sensibilité de détection au peroxyde d'hydrogène comparable aux micro-électrodes sans sous-couche isolante.

### Exemple 8 : mesure de PO₂

Des mesures de PO₂ ont été réalisées à l'aide de microélectrodes composées d'une fibre de carbone platinée par dépôt physique en phase vapeur, fabriquées selon la méthode décrite en Exemple 1. La Figure 1 montre les relations courant-PO₂ pour 6 électrodes issues de 6 lots différents, dans un tampon phosphate salin (PBS) à 0.01 M et pH 7.45 milieu standard comprenant des concentrations connues d'oxygène dissous mesurées en parallèle avec un système commercial Oxylab (Oxford Optronics, UK). La mesure d'oxygène dissous est effectuée en ampérométrie continue à -800 mV vs Ag/AgCl, par réduction de l'O₂. Comme le montre la Figure 1, le courant mesuré est proportionnel à la concentration d'oxygène dans le milieu de mesure.

Ces résultats montrent que les électrodes de détection électrochimique selon l'invention peuvent être utilisées comme capteurs de PO₂.

Il est bien entendu que les modes de réalisation qui ont été décrits ci-dessus ont été donnés à titre indicatif et non limitatif et que des modifications peuvent être apportées sans que l'on s'écarte pour autant du cadre de la présente invention.

### Références bibliographiques

Pernot, P., Mothet, J.-P., Schuvailo, O., Soldatkin, A., Pollegioni, L., Pilone, M., Adeline, M.-T., Cespuglio, R., Marinesco, S., 2008. Characterization of a yeast D-amino acid oxidase microbiosensor for D-serine detection in the central nervous system. Anal. Chem. 80, 1589-1597. doi:10.1021/ac702230w

Sakslund, H., Wang, J., 1995. Development and evaluation of glucose microsensors based on electrochemical codeposition of ruthénium and glucose oxidase onto carbon fiber microelectrodes. Journal of Electroanalytical Chemistry - J ELECTROANAL CHEM 397, 149-155. doi:10.1016/0022-0728(95)04152-0

Schuvailo, O.N., Dzyadevych, S.V., El'skaya, A.V., Gautier-Sauvigné, S., Csöregi, E., Cespuglio, R., Soldatkin, A.P., 2005. Carbon fibre-based microbiosensors for in vivo measurements of acetylcholine and choline. Biosens Bioelectron 21, 87-94. Wang, J., Rivas, G., Chicharro, M., 1997. Glucose microsensor based on electrochemical deposition of iridium and glucose oxidase onto carbon fiber electrodes. Journal of Electroanalytical Chemistry 439, 55-61. doi:10.1016/S0022-0728(97)00371-9

## Revendications

1. Procédé de fabrication d'une électrode de détection électrochimique, ledit procédé comprenant :
a) le dépôt d'une sous-couche isolante sur le filament support, ladite sous-couche isolante étant à base d'alumine,
b) le dépôt d'une sous-couche d'accroche sur la sous-couche isolante et,
c) le dépôt d'une couche métallique sur la sous-couche d'accroche,
lesdits dépôts étant réalisés par un procédé de dépôt sous vide.

2. Procédé selon la revendication 1, dans lequel le procédé de dépôt sous vide est un procédé de dépôt chimique en phase vapeur ou un procédé de dépôt physique en phase vapeur.

3. Procédé selon l'une des revendications 1 ou 2, dans lequel la couche métallique comprend au moins un métal noble, ledit métal noble pouvant être le platine, le ruthénium, le rhodium, le palladium ou leur combinaison.

4. Procédé selon la revendication 3, dans lequel la sous-couche d'accroche est à base de chrome, de titane ou de nitrure de titane.

5. Procédé selon l'une des revendications 1 à 4, dans lequel le filament support est une fibre de carbone.

6. Procédé selon l'une des revendications 1 à 5, comprenant, après le dépôt de la couche métallique, le dépôt d'un matériau écran apte à limiter la pénétration de molécules interférentes au contact du filament métallisé.

7. Procédé selon l'une des revendications 1 à 6, comprenant, après le dépôt de la couche métallique, le dépôt d'une couche enzymatique apte à catalyser la dégradation d'un analyte en générant un composé oxydable.

8. Procédé selon la revendication 7, dans lequel l'enzyme est de la classe des oxydases.

9. Électrode de détection électrochimique comprenant :
- un filament support revêtu d'une couche métallique déposée par un procédé de dépôt sous vide,
- une sous-couche isolante entre le filament support et la couche métallique, ladite sous-couche isolante étant à base d'alumine, et
- une sous-couche d'accroche entre la sous-couche isolante et la couche métallique.

10. Électrode selon la revendication 9, dans laquelle la couche métallique est revêtue d'une couche enzymatique apte à catalyser la dégradation d'un analyte en générant un composé oxydable, dans laquelle l'enzyme est de la classe des oxydases.

11. Électrode selon l'une des revendications 9 ou 10, comprenant, entre la couche métallique et la couche enzymatique, un matériau écran apte à limiter la pénétration de molécules interférentes au contact du filament métallisé.

12. Electrode selon l'une des revendications 9 à 11, dans laquelle :
le diamètre du filament n'excède pas 10 µm ;
l'épaisseur de la couche métallique n'excède pas 200 nm; et/ou
l'épaisseur de la sous-couche d'accroche n'excède pas 20 nm ; et/ ou
le diamètre total de l'électrode n'excède pas 15 µm.

13. Dispositif pour la détection électrochimique d'un analyte, ledit dispositif comprenant :
une électrode selon l'une des revendications 9 à 12 ;
un dispositif d'amplification de courant électrique ;
et, au moins l'un parmi :
un dispositif de conversion analogique/numérique ;
un logiciel d'acquisition et/ou d'exploitation des données de détection ;
une électrode de référence.

14. Utilisation de l'électrode de détection électrochimique selon l'une des revendications 9 à 12, caractéristisée en ce que la couche métallique est apte à catalyser l'oxydation d'un composé oxydable et/ou la réduction d'un composé réductible.

15. Utilisation selon la revendication 14, pour la détection de l'O₂.

16. Procédé de détection électrochimique d'un analyte dans un milieu de mesure, comprenant:
le placement d'une électrode selon l'une des revendications 9 à 12, dans le milieu de mesure;
l'application d'un potentiel de travail au biocapteur électrochimique;
la détection d'un courant d'oxydo-réduction indicateur de la présence et/ou de la concentration dudit analyte dans le milieu de mesure.

17. Procédé selon la revendication 16, dans lequel l'analyte est le glucose, le lactate, le glutamate, la choline, l'acétylcholine, l'adénosine, l'ATP, le cholestérol, la glycine, un D-amino acide ou l'O₂.

## Patentansprüche

1. Verfahren zur Herstellung einer Elektrode zur elektrochemischen Detektion, wobei das Verfahren umfasst:
a) Ablagerung einer Isoliergrundschicht auf das Trägerfilament, wobei die Isoliergrundschicht auf Basis von Aluminiumoxid gebildet wird,
b) Ablagerung einer Haftgrundschicht auf die Isoliergrundschicht, und
c) Ablagerung einer Metallschicht auf die Haftgrundschicht,
wobei die Ablagerungen mittels eines Vakuumabscheidungsverfahrens realisiert werden.

2. Verfahren nach Anspruch 1, wobei das Vakuumabscheidungsverfahren ein chemisches Gasphasenabscheidungsverfahren oder ein physikalisches Gasphasenabscheidungsverfahren ist.

3. Verfahren nach einem der Ansprüche 1 oder 2, wobei die Metallschicht mindestens ein Edelmetall enthält, wobei das Edelmetall Platin, Ruthenium, Rhodium, Palladium oder eine Kombination davon ist.

4. Verfahren nach Anspruch 3, wobei die Haftgrundschicht auf Basis von Chrom, Titan oder Titannitrid gebildet wird.

5. Verfahren nach einem der Ansprüche 1 bis 4, wobei das Trägerfilament eine Kohlenstofffaser ist.

6. Verfahren nach einem der Ansprüche 1 bis 5, umfassend, nach der Aufbringung der Metallschicht, die Aufbringung eines Abschirmungsmaterials, das in der Lage ist, das Eindringen störender Moleküle in Kontakt mit dem metallisierten Filament zu begrenzen.

7. Verfahren nach einem der Ansprüche 1 bis 6, umfassend, nach der Aufbringung der Metallschicht, die Aufbringung einer Enzymschicht, die in der Lage ist, den Abbau eines Analyten durch Erzeugung einer oxidierbaren Verbindung zu katalysieren.

8. Verfahren nach Anspruch 7, wobei das Enzym zur Klasse der Oxidasen gehört.

9. Elektrode zur elektrochemischen Detektion, umfassend:
- ein Trägerfilament, das mit einer Metallschicht beschichtet ist, die durch ein Vakuumabscheidungsverfahren abgelagert wurde,
- eine Isoliergrundschicht zwischen dem Trägerfilament und der Metallschicht, wobei diese Isoliergrundschicht auf Basis von Aluminiumoxid gebildet ist, und
- eine Haftgrundschicht zwischen der Isoliergrundschicht und der Metallschicht.

10. Elektrode nach Anspruch 9, wobei die Metallschicht mit einer Enzymschicht beschichtet ist, die in der Lage ist, den Abbau eines Analyten durch Erzeugung einer oxidierbaren Zusammensetzung zu katalysieren, wobei das Enzym der Klasse der Oxidasen angehört.

11. Elektrode nach einem der Ansprüche 9 oder 10, umfassend, zwischen der Metallschicht und der Enzymschicht, ein Abschirmungsmaterial, das in der Lage ist, das Eindringen störender Moleküle in Kontakt mit dem metallisierten Filament zu begrenzen.

12. Elektrode nach einem der Ansprüche 9 bis 11, wobei:
der Durchmesser des Filaments 10 µm nicht überschreitet;
die Dicke der Metallschicht 200 nm nicht überschreitet; und/oder
die Dicke der Haftgrundschicht 20 nm nicht überschreitet; und/oder der Gesamtdurchmesser der Elektrode 15 µm nicht überschreitet.

13. Vorrichtung für die elektrochemische Detektion eines Analyten, wobei die Vorrichtung umfasst:
eine Elektrode nach einem der Ansprüche 9 bis 12;
eine Vorrichtung zur Verstärkung des elektrischen Stroms;
und mindestens eines der folgenden Elemente:
eine Analog/Digital-Wandlungseinrichtung;
eine Software zur Erfassung und/oder Verarbeitung von Daten der Detektion;
eine Referenzelektrode.

14. Verwendung der Elektrode zur elektrochemischen Detektion nach einem der Ansprüche 9 bis 12, **dadurch gekennzeichnet, dass** die Metallschicht in der Lage ist, die Oxidation einer oxidierbaren Zusammensetzung und/oder die Reduktion einer reduzierbaren Zusammensetzung zu katalysieren.

15. Verwendung nach Anspruch 14 für die Detektion von O₂.

16. Verfahren zur elektrochemischen Detektion eines Analyten in einer Messumgebung, umfassend:
Platzieren einer Elektrode nach einem der Ansprüche 9 bis 12 in die Messumgebung;
Anlegen eines Arbeitspotentials an den elektrochemischen Biosensor;
Erfassen eines Redoxstroms, der das Vorhandensein und/oder die Konzentration des Analyten in der Messumgebung anzeigt.

17. Verfahren nach Anspruch 16, wobei der Analyt Glucose, Lactat, Glutamat, Cholin, Acetylcholin, Adenosin, ATP, Cholesterin, Glycin, eine D-Aminosäure oder O₂ ist.

## Claims

1. Method for manufacturing an electrode for electrochemical detection, said method comprising:
a) depositing an insulating sub-layer on the support filament, said insulating sub-layer being based on alumina,
b) depositing an adhesion sub-layer on the insulating sub-layer and,
c) depositing a metallic layer on the adhesion sub-layer,
said depositions being carried out by a vacuum deposition method.

2. Method according to claim 1, in which the vacuum deposition method is a chemical vapour deposition method or a physical vapour deposition method.

3. Method according to one of claims 1 or 2, in which the metallic layer comprises at least one noble metal, it being possible for said noble metal to be platinum, ruthenium, rhodium, palladium or a combination thereof.

4. Method according to claim 3, in which the adhesion sub-layer is based on chromium, titanium or titanium nitride.

5. Method according to one of claims 1 to 4, in which the support filament is a carbon fibre.

6. Method according to one of claims 1 to 5, comprising, after deposition of the metallic layer, depositing a shielding material capable of limiting the penetration of interfering molecules on contact with the metallized filament.

7. Method according to one of claims 1 to 6, comprising, after deposition of the metallic layer, depositing an enzymatic layer capable of catalysing the degradation of an analyte by generating an oxidizable compound.

8. Method according to claim 7, in which the enzyme is of the oxidase class.

9. Electrode for electrochemical detection comprising:
- a support filament coated with a metallic layer deposited by a vacuum deposition method,
- an insulating sub-layer between the support filament and the metallic layer, said insulating sub-layer being based on alumina, and
- an adhesion sub-layer between the insulating sub-layer and the metallic layer.

10. Electrode according to claim 9, in which the metallic layer is coated with an enzymatic layer capable of catalysing the degradation of an analyte by generating an oxidizable compound, in which the enzyme is of the oxidase class.

11. Electrode according to one of claims 9 or 10, comprising, between the metallic layer and the enzymatic layer, a shielding material capable of limiting the penetration of interfering molecules on contact with the metallized filament.

12. Electrode according to one of claims 9 to 11, in which:
the diameter of the filament does not exceed 10 µm;
the thickness of the metallic layer does not exceed 200 nm; and/or
the thickness of the adhesion sub-layer does not exceed 20 nm; and/or
the total diameter of the electrode does not exceed 15 µm.

13. Device for electrochemical detection of an analyte, said device comprising:
an electrode according to one of claims 9 to 12;
a device for amplifying an electric current;
and at least one from:
a device for analogue/digital conversion;
software for acquiring and/or processing detection data;
a reference electrode.

14. Use of the electrode for electrochemical detection according to one of claims 9 to 12, **characterized in that** the metallic layer is capable of catalysing the oxidation of an oxidizable compound and/or the reduction of a reducible compound.

15. Use according to claim 14, for detecting O₂.

16. Method for electrochemical detection of an analyte in a measurement medium, comprising:
placing an electrode according to one of claims 9 to 12 in the measurement medium;
applying a working potential to the electrochemical biosensor;
detecting an oxidation-reduction current indicating the presence and/or the concentration of said analyte in the measurement medium.

17. Method according to claim 16, in which the analyte is glucose, lactate, glutamate, choline, acetylcholine, adenosine, ATP, cholesterol, glycine, a D-amino acid or O₂.
